# NEUE EUROPÄISCHE PATENTSCHRIFT

(11) **EP 0 525 502 B2**
(45) Veröffentlichungstag und Bekanntmachung des Hinweises auf die Entscheidung über den Einspruch: **10.08.2005**
(45) Hinweis auf die Patenterteilung: 09.10.1996
(21) Anmeldenummer: 92111947.5
(22) Anmeldetag: 14.07.1992
(51) Int. Cl.: A61K 39/395, A61L 2/00, C07K 1/20

(54) **Verfahren zur Herstellung von virusinaktivierten Immunglobulinlösungen**
Method for the preparation of virus-inactivated immunoglobuline solutions
Procédé pour la fabrication de solutions contenant des immunoglobulines et des viruses inactivés

(30) Priorität: 02.08.1991 DE 4125625
(43) Veröffentlichungstag der Anmeldung: 03.02.1993
(73) Patentinhaber: OCTAPHARMA AG, 8866 Ziegelbrücke (CH)
(72) Erfinder: Gehringer, Werner, A-1160 Wien (AT); Selosse, Patrick, F-33200 Bordeaux (FR)
(74) Vertreter: Werner, Hans-Karsten, Dr.Dipl.-Chem.

(56) Entgegenhaltungen:
- EP-A- 0 131 740
- EP-A- 0 239 859
- EP-A- 0 322 786
- EP-A- 0 366 946
- Colin T.Mant et al "High performance liquid Chromatography of peptides and Proteins; Seporation, Analysis and Confirmation", CRC Press Boca Raton
- Milton, T.W.Hern "HPLC of Proteins, peptides and Polynucleotides, contemporary topics and applications", VCH Verlag, Heidelberg

## Beschreibung

Gegenstand der Erfindung ist ein Verfahren zur Herstellung virusinaktivierter Immunglobulinlösungen, die zur intravenösen Injektion geeignet sind.

Immunglobuline sind humorale Glykoproteine, die bei der Elektrophorese der Plasma- bzw, Serumproteine in der sogenannten Gamma-Fraktion wandern und daher früher als Gamma-Globuline bezeichnet wurden.

Immunglobuline werden wegen ihres hohen Gehalts an Antikörpern zur Prophylaxe und Therapie von Infektionen verwendet.

Es ist bekannt, Immunglobuline sowohl für die intramuskuläre als auch subkutane Applikation herzustellen. Eine vielfach angewendete Herstellungsmethode ist die sogenannte Cohn-Oncley-Fraktionierung, auch 6/9-Methode genannt (Cohn et al., J. Am. Chem. Soc. 68, 459 (1946); Oncley et al.; J. Am. Chem. Soc. 71, 541 (1949).

Dieses Herstellungsverfahren hat jedoch den Nachteil, daß es zu einer hochviskosen Lösung, die nur intramuskulär oder subkutan applizierbar ist, mit hohen Antikörperkonzentrationen in relativ geringen Volumina führt. Das erhaltene Produkt ist zwar bei 4°C stabil, es kann jedoch Proteolyse durch Plasminverunreinigungen auftreten. Weiterhin können IgA- und IgG-Dimere vorhanden sein, die bei der Applikation zu einer anaphylaktischen Reaktion der Patienten führen können (Ullmann, Enzyklopädie of Industrial Chemistry, 1989, A14, Seiten 93, 94).

Aus diesem Grunde sind intravenös applizierbare Immunglobuline entwickelt worden, die beim Patienten eine bessere Verträglichkeit zeigen. Diese werden aus der sogenannten Fraktion 3 oder auch Cohn-Fraktion 2 (Cohn et al. a. a. O.) bei pH4 unter Verwendung von Polyethylenglycol, einer anschließenden Ethanolfällung. Ultra- oder Diafiltration und lonenaustauscherchromatographie hergestellt. Das auf diese Art erhaltene Immunglobulin wird mit Mono- oder Disacchariden stabilisiert.

Ein solches verbessertes Verfahren wird in der EP 0 073 371 beschrieben.

Ausgehend von der Fraktion 3 (Cohn et al. a. a. O.) wird nach Lösen und Einstellung des pH-Wertes auf pH4 eine Ultrafiltration und eine Diafiltration vorgenommen. Anschließend wird das erhaltene Filtrat zu einem Proteingehalt von 5 Gew.-% konzentriert und der Alkoholgehalt auf 8 Gew.-% reduziert. Nachdem die so erhaltene Immunglobulinlösung auf einen Proteingehalt von 8% aufkonzentriert ist, erhält man eine klare wasserartige Lösung mit einer Ionenstärke von 0,01 und einem pH-Wert von 4,2. Die Tonizität der Lösung wird mit 10 Gew.-% Maltose bei einem Proteingehalt von 5 Gew.-% eingestellt. Anschließend wird sterilfiltriert und lyophilisiert. Das lyophilisierte Material wird vor der Injektion in geeigneten Medien gelöst.

Ein großer Nachteil der so hergestellten lmmunglobuline zur intravenösen Anwendung ist, daß sie vor Gebrauch erst in geeigneten Medien gelöst werden müssen und nur in lyophilisierter Form lagerfähig sind.

Ein weiterer Nachteil liegt darin, daß bei Anwendung dieser Immunglobuline Viren auf den Patienten übertragen werden können, da während des Herstellungsverfahrens keine Virusinaktivierung stattfindet. So wurde nach intravenöser Immunglobulingabe über Hepatitis-Erkrankungen und HIV-Infektionen berichtet (Ullmanns Encycl. of Ind. Chem., Vol. A14, 1989, pages 102, 103).

EP-A1-366946 beschreibt die Entfernung von nichtionischen Detergentien, die zur Virusinaktivierung verwendet werden, aus biologischen Flüssigkeiten, wie Immunoglobulin. Dabei wird eine hydrophobe Interaktionschromatographie angewandt

EP-A2-239859 beschreibt die Entfernung von virusinaktivierenden Detergentien aus biologischen Flüssigkeiten, wie Immunoglobulin mit Hilfe einer Extraktion mit Ölen.

Das technische Problem der Erfindung war es daher, ein Verfahren zu entwickeln, das zu einem Produkt führt, welches so stabil ist, daß es selbst ohne Lyophilisierung direkt als Injektionslösung hergestellt und gelagert werden kann und bei dessen Applikation keine Virusübertragungen auf den Patienten auftreten.

Das technische Problem der Erfindung wird durch ein Verfahren gelöst, wobei das Immunglobulin mit nichtionischen. Tensiden behandelt wird, die anschließend durch Festphasenextraktion an hydrophoben Materialien entfernt werden und nach der Behandlung mit nichtionischen Tensiden mit biologisch kompatiblen Pflanzenölen extrahiert diese abgetrennt werden, und dann die Festphasenextraktion durch geführt wird.

Als nichtionische Tenside werden insbesondere TNBP und/oder Triton X 100 verwendet. Der pH-wert der Lösung beträgt vorzugsweise 5,0 bis 5,5.

Als Pflanzenöle werden bevorzugt Rizinusöl oder Sojaöle verwendet.

Die anschließende Festphasenextraktion erfolgt in bevorzugter Weise mit octadecylderivatisierten Stoffen, die auch für die Umkehrphasenchromatographie verwendet werden. In einer besonders bevorzugten Ausführungsform erfolgt die Festphasenextraktion durch eine Umkehrphasenchromatographie an Octadecyl-(C-18)-Harz.

Dem fertigen Produkt kann nach der Festphasenextraktion ein Disaccharid zur Stabilisierung zugegeben werden. Die fertige Lösung wird dann ein oder mehrmals sterilfiltriert.

Weiterhin kann die Immunglobulinlösung vor der Virenaktivierung und/oder nach der Sterilfiltration einer Ultra- und Diafiltration unterzogen werden.

Im folgenden wird das erfindungsgemäße Herstellungsverfahren im einzelnen beschrieben. Zunächst wird, wie aus dem Stand der Technik bekannt, die sogenannte Cohn-Fraktion II in Wasser gelöst, bis eine vollständige klare Lösung erhalten wird. Die Lösung wird dann auf einen pH-Wert von 4,0 bis 5,0, vorzugsweise 4,5, eingestellt und zur Entfernung von Verunreinigungen filtriert.

Anschließend wird mit der Lösung eine Ultrafiltration durchgeführt und die Lösung ankonzentriert. Die Ausschlußgrenze der Ultrafiltration ist 30.000 Dalton. In diesem Schritt werden insbesondere Verunreinigungen mit niedrigen Molekulargewichten entfernt. Daran anschließend wird eine Diafiltration zur Entfernung von Ionen vorgenommen, woran sich die eigentliche Virusinaktivierung abschließt.

Hierzu wird die Lösung zunächst abgekühlt auf 4 bis 8 °C und der pH-Wert auf 5,0 bis 5,5, vorzugsweise 5,3, eingestellt. Es werden dann nichtionische Tenside, vorzugsweise TNBP und/oder Triton X 100, zugegeben und diese Lösung dann mehrere Stunden gerührt. Im Anschluß daran wird eine Pflanzenölextraktion vorgenommen. Hierbei werden der Lösung 5 Gew.-% Pflanzenöl zugegeben, die Lösung anschließend auf Raumtemperatur gebracht und mit dem Pflanzenöl verrührt. Der anschließenden Phasentrennung wird eine Filtration angeschlossen.

Die Lösung wird dann auf eine C 18-Säule gegeben und chromatographiert. Nach der Chromatographie erfolgt eine pH-Werteinstellung auf pH 4. Zur Einstellung der Tonizität wird Maltose zugegeben. Nach der anschließenden Sterilfiltration wird eine Stabilitätsprüfung in der so erhaltenen Lösung vorgenommen, indem diese mindestens 22 Stunden bei 37°C aufbewahrt wird. Zeigt die Lösung Trübungen, ist sie nicht verwendbar. Zeigt die Lösung in dieser Zeit keine Trübung, wird der pH-Wert auf 5,0 bis 5,5, vorzugweise 5,3, eingestellt, eine weitere Ulra- und Diafiltration vorgenommen und die so erhaltene Lösung nach Zugabe von Maltose auf einen Proteingehalt von 50 g/l eingestellt Arischließend wird eine weitere Sterilfiltration vorgenommen und direkt in Infusionsflaschen abgefüllt.

Das so erhaltene Produkt kann direkt intravenös injiziert werden und ist frei von Viren.

Das folgende Ausführungsbeispiel soll das erfindungsgemäße Verfahren näher erläutern.

### Ausführungsbeispiel

Die Cohn-Fraktion II wird mit der sechsfachen Menge Wasser gelöst und so lange gerührt, bis eine klare Lösung erhalten wird. Anschließend wird der pH-Wert mit 0,5 normaler HCl auf 4,5 eingestellt. Es schließt sich zunächst eine Ultrafiltration an, bei der die Lösung auf 90 g/l ankonzentriert wird. Als Membrantyp wird Novasette 30 K verwendet. Daran anschließend wird mit der fünffachen Menge Wasser verdünnt und bei 0,3 bis 0,5 bar eine Diafiltration vorgenommen. Nach dieser Diafiltration wird die diafiltrierte Lösung auf 70 g/l Proteingehalt eingestellt. Die Lösung wird auf 4 bis 8 °C abgekühlt und auf einen pH-Wert von 5,3 mittels 0,1 normaler Natronlauge eingestellt. Anschließend wird 0,3 Gew.-% TNBP und 1 Gew.-% Triton X 100 in die Lösung gegeben und kräftig gerührt. Nach etwa 4 Stunden bei 4 bis 8 °C erfolgt eine weitere Zugabe von 5 Gew.-% Rizinusöl. Es wird dann eine Ölextraktion bei 15 °C vorgenommen. Die entstehenden Phasen werden getrennt und es wird anschließend mit einem Cuno-Schichtenfilter filtriert. Danach wird die Lösung auf eine C 18-Säule gegeben, die mit octadecylderivatisierten Stoffen beladen ist. Die Lösung wird dann auf pH 4 eingestellt und 100 g/l Maltose zugegeben. Nachfolgend wird eine Sterilfiltration vorgenommen und die sterilfiltrierte Lösung zwischen 22 und 24 Stunden bei 37 °C aufbewahrt. Die anschließend klare Lösung wird auf einen pH-Wert von 5,3 mittels 0,1 normaler Natronlauge eingestellt. Es erfolgt wiederum eine Ultra- und eine Diafiltration, danach eine Maltosezugabe von 100 g/l und die Einstellung der Lösung auf einen Proteingehalt von 50 g/l. Nach der folgenden Sterilfiltration wird die Lösung in 50 ml Infusionsflaschen, die sterilisiert und silikonisiert sind, abgefüllt, mit Stopfen verschlossen und verbördelt.

## Patentansprüche

1. Verfahren zur Herstellung von zur intravenösen Applikation geeigneten virusinaktivierten Immunglobulinlösungen, wobei das Immunglobulin mit nichtionischen Tensiden behandelt wird, die anschließend durch Festphasenextraktion an hydrophoben Materialien entfernt werden, **dadurch gekennzeichnet, daß** nach der Behandlung mit nichtionischen Tensiden mit biologisch kompatiblen Pflanzenölen extrahiert wird, diese abgetrennt werden und dann die Festphasenextraktion durchgeführt wird.

2. Verfahren nach Anspruch 1, **dadurch gekennzeichnet, daß** der pH-Wert der Lösung 5,0 bis 5,5 beträgt.

3. Verfahren nach Anspruch 1 oder 2, **dadurch gekennzeichnet, daß** als nichtionische Tenside TNBP (Tri-n-butyl-phosphat) und/oder Triton X 100 verwendet werden.

4. Verfahren nach Anspruch 1 bis 3, **dadurch gekennzeichnet, daß** als Pflanzenöle Rizinusöl und/oder Sojaöl verwendet werden.

5. Verfahren nach den Ansprüchen 1 bis 4, **dadurch gekennzeichnet, daß** die Festphasenextraktion mit octadecylderivatisierten Stoffen, die auch für die Umkehrphasenchromatographie verwendet werden, durchgeführt wird.

6. Verfahren nach den Ansprüchen 1 bis 5, **dadurch gekennzeichnet, daß** die Festphasenextraktion durch C 18 Umkehrphasenchromatographie erfolgt.

7. Verfahren nach den Ansprüchen 1 bis 6, **dadurch gekennzeichnet, daß** der Lösung nach der Festphasenextraktion ein Disaccharid zugegeben wird.

8. Verfahren nach Ansprüchen 1 bis 7, **dadurch gekennzeichnet, daß** die fertige Lösung ein- oder mehrmals sterilfiltriert wird.

9. Verfahren nach den Ansprüchen 1 bis 8, **dadurch gekennzeichnet, daß** die Immunglobulinlösung vor der Virusinaktivierung und/oder nacti der Sterilfiltration einer Ultra- und Diafiltration unterzogen wird.

10. Verfahren nach den Ansprüchen 1 bis 9, **dadurch gekennzeichnet, daß** die nach der Festphasenextraktion oder der Disaccharidzugabe eine Stabilitätsprüfung der hergestellten Lösung vorgenommen wird.

## Claims

1. A method for the preparation of virus-inactivated immunoglobulin solutions suitable for intravenous application wherein said immunoglobulin is treated with non-ionic surfactants which are subsequently removed by solid-phase extraction on hydrophobic materials, **characterized in that** after said treatment with non-ionic surfactants extraction is performed with biologically compatible vegetable oils, these are separated off, and then the solid phase extraction is performed.

2. The method according to claim 1, **characterized in that** the pH value of said solution is from 5.0 to 5.5.

3. The method according to claim 1 or 2, **characterized in that** TNBP (tri-n-butyl phosphate) and/or Triton X 100 are used as said non-ionic surfactants.

4. The method according to claims 1 to 3, **characterized in that** castor oil and/or soybean oil are used as said vegetable oils.

5. The method according to claims 1 to 4, **characterized in that** said solid-phase extraction is performed with octadecyl derivatized materials which are also being used for reversed phase chromatography.

6. The method according to claims 1 to 5, **characterized in that** said solid-phase extraction is performed by C₁₈ reversed phase chromatography.

7. The method according to claims 1 to 6, **characterized in that** after said solid-phase extraction a disaccharide is added to the solution.

8. The method according to claims 1 to 7, **characterized in that** the finished solution is sterile filtrated once or several times.

9. The method according to claims 1 to 8, **characterized in that** prior to said virus inactivation and/or after said sterile filtration said immunoglobulin solution is subjected to ultrafiltration and diafiltration.

10. The method according to claims 1 to 9, **characterized in that** after said solid-phase extraction or after said addition of disaccharide the solution prepared is subjected to a stability test.

## Revendications

1. Procédé de préparation de solutions d'immunoglobuline avec un virus inactivé, appropriées à une administration par voie intraveineuse, selon lequel on traite l'immunoglobuline avec des agents tensio-actifs non-ioniques qui, ensuite, sont éliminés par extraction en phase solide sur des matériaux hydrophobes, **caractérisé en ce que**, après ce traitement avec des agents tensio-actifs non-ioniques, on extrait l'immunoglobuline avec des huiles végétales biologiquement acceptables on sépare celles-ci et, ensuite on procède à l'extraction en phase solide.

2. Procédé selon la revendication 1, **caractérisé en ce que** le pH de la solution est de 5,0 à 5,5.

3. Procédé selon la revendication 1 ou 2, **caractérisé en ce qu'**on utilise, comme agents tensio-actifs non-ioniques, du TNBP (phosphate de tri-n-butyle) et/ou du Triton X 100.

4. Procédé selon l'une des revendications 1 à 3, **caractérisé en ce qu'**on utilise, comme huiles végétales, l'huile de ricin et/ou l'huile de soja.

5. Procédé selon les revendications 1 à 4, **caractérisé en ce qu'**on effectue l'extraction en phase solide avec des substances dérivées du radical octadécyle, qu'on utilise aussi pour la chromatographie à phases inversées.

6. Procédé selon les revendications 1 à 5, **caractérisé en ce qu'**on effectue l'extraction en phase solide par chromatographie à phases inversées sur une colonne C 18.

7. Procédé selon les revendications 1 à 6, **caractérisé en ce qu'**on ajoute un disaccharide à la solution après l'extraction en phase solide.

8. Procédé selon les revendications 1 à 7, **caractérisé en ce qu'**on soumet la solution finie à une ou plusieurs opérations de filtration dans des conditions stériles.

9. Procédé selon les revendications 1 à 8, **caractérisé en ce qu'**on soumet la solution d'immunoglobuline à une ultrafiltration et à une diafiltration avant l'inactivation du virus et/ou après la filtration dans des conditions stériles.

10. Procédé selon les revendications 1 à 9, **caractérisé en ce que**, après l'extraction en phase solide ou après l'addition du disaccharide, on contrôle la stabilité de la solution préparée.
